# EUROPEAN PATENT SPECIFICATION

(11) **EP 0 428 486 B1**
(45) Date of publication and mention of the grant of the patent: **21.09.1994**
(21) Application number: 90810872.3
(22) Date of filing: 13.11.1990
(51) Int. Cl.: A61K 47/48

(54) **Polymyxin conjugates**
Polymyxinkonjugate
Conjugués de polymyxine

(30) Priority: 15.11.1989 US 437487
(43) Date of publication of application: 22.05.1991
(73) Proprietor: SANDOZ LTD., 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Handley, Dean Allen, Mountain Lakes, NJ. 07046 (US); Lake, Philip, Parsippany, NJ. 07950 (US)

(56) References cited:
- EP-A- 0 147 761
- FR-A- 2 342 740
- FILE SERVER STN, Karlsruhe, FILE BIOSIS, abstract no. 85:421296, accession no.Biological abstract 80:91288; T. KITAGAWA et al.: "Sensitive enzyme immunoassayof colistin and its application to detect residual colistin in rainbow trouttissue"

## Description

The invention relates to novel polymyxin conjugates and to methods of preparing and using them.

Endotoxins or lipopolysaccharides are structural molecules derived from the cell walls of the Gram-negative bacteria. When introduced into the bloodstream, they can interfere with the regulation of body temperature and cause fever. They also have a toxic effect, leading to cardiac, pulmonary and kidney failure. Endotoxin-related diseases are a leading cause of death among those patients in intensive care units.

Unique among antibiotics is the ability of polymyxins, especially of polymyxin B (PMB) to neutralize endotoxin, accomplished by binding to the lipid A region of the endotoxin molecule. Polymyxin B from Bacillus polymyxa (B. aerosporus) is a highly charged amphiphilic cyclic peptidolipid. It is also useful in combating various fungal infections, especially those arising in immunocompromised individuals.

However, PMB has some properties which renders it less than an ideal antibiotic. First, it has a short half-life in the body, requiring repeated dosages in order to be effective. Secondly, as it passes through the kidney it can cause extensive damage. Thirdly, at high doses it possesses neurotoxic properties which cause respiratory paralysis.

Previously, researchers have conjugated polymyxin to immobile or fixed molecules. See e.g. Issekutz, J. Immunol. Methods 61 (1983) 275-281, describing the binding of PMB to Sepharose. These conjugates, while useful in purification techniques, are not suitable for in vivo therapeutic use.

One approach to achieve pharmacological activity, increased duration, or decreased organ toxicity has involved the conjugation of drugs to large molecular weight macromolecules such as dextran, polyethylene glycol or polyvinylpyrrolidine. Attempts in this area of polymer conjugation have been met with only limited success, however. For example, the conjugated form of procainamide (an antiarrythmic drug) was less active and exhibited a shorter half-life than native procainamide (Schacht et al., Ann. NY Acad. Sci. (1985) 446 199-211). Similarly, a prostaglandin analog, B245, linked to a carrier, was less effective (by several log orders) than the native molecule [Bamford et al., Bioch. Biophys. Acta 886 (1986) 109-118]. Reductions in biological potency have also been described for conjugated forms of kallikrein, aprotinin, bradykinin [Odya et al., Biochem. Pharmacol. 27 (1978) 173-179] and the anti-tumor drugs daunorubicin [Hurwitz et al., J. Appl. Biochem. 2 (1980) 25-35] and mitomycin C [Takakura et al., Cancer Res. 44 (1984) 2505-2510]. Conjugated enzymes also suffer a reduction in biological activity due to steric hindrance and reduced substrate accessibility [Blomhoff et al., Biochem. Biophys. Acta 757 (1983) 202-208; Marshall et al., J. Biol. Chem. 251 (1976) 1081-1087; Foster, Experientia 31 (1975) 772-3; Wileman et al., J. Pharm. Pharmacol. 35 (1983) 762-765]. There are, however, some examples of improvements in circulatory half-life after conjugation [Wileman, supra; Kaneo, Chem. Pharm. Bull. 37 (1989) 218-220].

It would be desirable to develop a form of polymyxin which would stay in the blood stream longer and/or does not have neuro- or nephrotoxicity at therapeutic doses.

The present invention relates to polymyxin-carrier conjugates which are soluble in water, e.g. polymyxin A-, B- or E-carrier conjugates, particularly PMB-carrier conjugates. They are indicated for use in neutralizing endotoxin. They are an improvement over the administration of native polymyxin because they are more effective and are less toxic.

As used herein the following definitions apply:
- LD₀ is the highest non-toxic dose of PMB or its conjugate;
- LD₁₀₀ is the dose of PMB or its conjugated form which results in 90-100% lethality when injected into a normal test animal;
- PD₁₀₀ is the dose of polymyxin B or its conjugated form which, when injected into a hypersensitized test animal, results in at least 95 % survival;
- Therapeutic Index (TI) is calculated by dividing LD₁₀₀ by PD₁₀₀.

As defined herein a conjugate is water-soluble when it has a solubility in water of at least 0.5 mg/ml at 25°C, preferably of at least 1 mg/ml at 25°C. Such conjugates are water-soluble and injectable, non-matrix, colloid, not cross-linked, non-macromolecular, and/or non-particulate.

The carrier which can be conjugated to polymyxin is chosen from molecules which can form water-soluble conjugates and which are uniform, non-toxic, non-carcinogenic, non-irritating and non-immunogenic. It is normally a biopolymer. Such carriers include polysaccharides such as dextran or hydroxy ethyl starch (HES), proteins such as albumin, and polymers such as polyvinyl pyrrolidone, polyethylene glycol and polyvinyl alcohol. Dextran is the most preferred.

The size of the biopolymer portion, i.e. the carrier part of the conjugate may vary. Typically it will range from a molecular weight of 25,000 to 500,000, preferably from 50,000 to 300,000, more preferably from 79,000 to 200,000. The size of the biopolymer chosen can significantly contribute to the duration of the conjugate's effective time of circulation in the blood stream. Generally, the larger the biopolymer, the longer the conjugate will stay in the circulation. Thus the size of the biopolymer can be adjusted to result in a conjugate whose time of duration in the body corresponds to a predetermined time.

The conjugates of the invention may be prepared in conventional manner.

Taking polymyxin B as an illustration, this has five γ-amino groups which bind to the bacterial endotoxin. At least one γ-amino group can be used to securely bind the PMB to the carrier, but all five sites cannot be used for this purpose, or the conjugate will lack endotoxin-neutralizing activity. Various chemical reactions can be used to conjugate polymyxin to these carriers. The number of PMB molecules bound per molecule of polymer can be influenced by varying the ratios of reactants during the coupling reaction. Generally the conjugate will contain between 1 PMB : 15 biopolymer to 200 PMB : 1 biopolymer, more preferably the conjugate will have from 1-10 PMB : 1 biopolymer, and even more preferably from 1-3 PMB : 1 biopolymer on a molar basis. The conjugates with high PMB : biopolymer ratios seem to possess greater potency than conjugates with lower ratios.

One method of making a polymyxin-carrier conjugate, more specifically, a PMB-dextran conjugate, is through carbamate linkage (method A). PMB-dextran conjugates made in this fashion (detailed in Example 1, below) are found to retain the anti-endotoxin activity of native PMB, and in addition are found to be devoid of the acute neurotoxicity exhibited by native PMB. These conjugated forms also show a 2-5 fold improved therapeutic index by decreasing chronic toxicity over that of native PMB.

A second method of making conjugated polymyxin dextran is through an amine bond (method B). PMB-dextran conjugates made in this manner (detailed in Example 2, below) retain the same general anti-endotoxin activity or are more active than native PMB. These conjugated forms are completely devoid of any acute neurotoxicity seen by native PMB and exhibit a 33-fold improvement of the therapeutic index by reducing chronic toxicity over that seen with native PMB. In particular, one PMB-dextran conjugate is devoid of any measurable toxicity at the doses tested and retains good anti-endotoxin activity, resulting in an 80-fold improvement in the therapeutic index.

Further, it has been surprisingly found that conjugates produced by this method and "rapidly purified" exhibit a 60- to 120-fold improvement in TI. "Rapidly purified" as used throughout the specification and claims means that the material is purified from unbound PMB using molecular sieving chromatography, de-salting gels, or Amicon^{R} ultrafiltration, occurs for approximately 12-24 hours, preferably approximately 18 hours (as opposed to an extended dialysis time of 7-10 days). Thus conjugates produced by these methods and rapidly purified comprise another and preferred aspect of this invention.

The polymyxin-carrier conjugate obtained from either of the two methods outlined above is, however, difficult to purify. Initially, a 10-day extended dialysis was tried in order to separate native PMB from the conjugate. Although the protein level inside the dialysis bag reached an asymptote, and gel permeation column chromatography showed a single large molecular weight species, the material still contained unbound PMB. Even when pressure dialysis (Amicon^{R}) filtration was substituted for extended dialysis the results were the same. Conjugates purified in this manner were equipotent to PMB, but still retained 1/3 to 1/5 the toxicity of PMB. While not intending to be bound by theory, it appears that there is some chemical association between the "free" PMB and the conjugate which renders its separation difficult. Upon injection in the animal the "free" PMB dissociates itself and causes toxicity problems.

The following procedure (method C) (detailed in Example 4, below) obviates the above difficulties. Polymyxin-dextran conjugates such as PMB-dextran conjugates are precipitated in a lower alcohol, preferably methanol, followed by centrifugal collection and ultrasonic resolubilization. This procedure is repeated, normally at least three times, and preferably 7 to 10 times and the resulting material is evaluated for purity, e.g. by gel permeation chromatography and reverse-phase high pressure liquid chromatography (RP-HPLC). If protein is detected by either method, the precipitation-resolubilization procedure is repeated. Conjugate purified in this manner is referred to herein as "ultra-pure" polymyxin-conjugate and generally can be described as having less than 20 µg unconjugated polymyxin per 1 mg total protein, and preferably less than 10 µg unconjugated polymyxin per 1 mg total protein.

A further aspect of the invention is thus a process for the preparation of a water-soluble polymyxin-dextran conjugate comprising
a) appropriately conjugating polymyxin to dextran to form a water-soluble polymyxin-dextran conjugate;
b) precipitating the resultant conjugate in a lower alcohol;
c) resuspending the conjugate; and
d) repeating steps b) and c) until the conjugate is substantially free from unbound polymyxin.

"Substantially free from unbound polymyxin" is herewith defined as a ratio of conjugate to unbound polymyxin of at least 95 : 1, preferably of at least 99 : 1 on a molar basis. The lower alcohol is e.g. of 1 to 10 carbon atoms, it preferably is of 1 to 4 carbon atoms, e.g. n-butanol or isopropanol, more preferably methanol or ethanol. The polymyxin preferably is PMB.

Ultra-pure conjugate has virtually no toxicity and as a result an LD₁₀₀ cannot be established for it. At a dose of 100 mg/kg no toxicity is seen. At higher concentrations the solution is too viscous to inject. This is in contrast to native PMB which has an LD₀ i.v. of 5 mg/kg and an LD₁₀₀ i.v. of 9.5 mg/kg. Thus the Therapeutic Index of ultra-pure PMB conjugate is over 1000-fold higher than native PMB, due to decreased toxicity.

A second advantage in using the conjugate rather than native polymyxin is that the conjugate has a longer duration of activity and thus can be used prophylactically. Thus one aspect of the invention is the prevention of diseases caused by the presence of bacterial endotoxin by administering to a subject in need of such prophylactic treatment a prophylactically-effective amount of a water-soluble conjugate of polymyxin, preferably PMB and a carrier, particularly dextran, and a pharmaceutically acceptable further carrier or diluent as appropriate, e.g. an inert solution. As the conjugate has virtually no toxicity it can be prophylactically administered on a routine basis to patients who might be susceptible to septic shock, such as those dependent upon liquid food for long-term nourishment.

The invention also concerns a pharmaceutical composition comprising a water-soluble polymyxin-carrier conjugate together with further pharmaceutically acceptable carriers or diluents as appropriate, e.g an inert solution.

The invention also concerns a process for the preparation of a pharmaceutical composition comprising mixing a water-soluble polymyxin-carrier conjugate with further pharmaceutically acceptable carriers or diluents as appropriate, e.g. an inert solution.

The invention is thus indicated for use in the treatment of diseases caused by the presence of bacterial endotoxin comprising administering to a subject in need of such treatment an endotoxin-neutralizing amount of a water-soluble conjugate of polymyxin, particularly PMB and a carrier, particularly dextran, and an inert solution. As polymyxin is also effective im combating bacterial or fungal infections, this invention is also indicated for use in treating bacterial or fungal infections comprising administering to a subject in need of such treatment a bacteriocidally- or fungicidally-effective amount of a water-soluble conjugate of polymyxin and a carrier, particularly dextran, and an inert solution.

The polymyxin-carrier conjugate of the invention can be used in a manner consistent with the use of polymyxin itself, i.e. it can be used alone as an antibiotic for bacterial or fungal infections or combined with other bacteriocidal agents and/or anti-inflammatory agents. It may be administered, e.g. intramuscularly, intravenously, intrathecally, subconjunctivally or topically. Thus formulations for intramuscular injections typically comprise an effective amount of PMB-conjugate in sterile water, physiological saline or approximately 1 % procane·HCl. Intravenous formulations typically comprise an effective amount of PMB-conjugate in 5 % dextrose and sterile water. Intrathecal formulations typically comprise an effective amount of PMB-conjugate in physiologic saline. For topical ophthalmic use, an effective amount can be mixed with water or physiological saline, and optionally glycerine, and cupric sulfate for eye drops, or it may be made into an ointment or suspension. Creams, for topical applications, especially for burned areas, typically comprise an effective amount of polymyxin-conjugate in a base of inactive ingredients such as liquid petrolatum, propylene glycol, polyoxyethylene, polyoxypropylene and emulsifying wax.

The amount of polymyxin-conjugate to be used is based on the amount of native polymyxin which would typically be prescribed for a particular patient (taking into account such factors as the condition being treated and age and weight of the patient) and the activity of the particular polymyxin-conjugate used. Often, a dosage reduction of up to 50 % compared to native polymyxin can be realized due to the effective increased activity of the polymyxin conjugate, its reduced toxicity and its increased duration of activity.

The invention is illustrated by reference to the following non-limiting Examples. All temperatures are in degrees Centigrade.

### Example 1: Chemical conjugation of PMB to dextran

### (Method A; carbamate linkage)

2 g dextran (79,000 or 200,000 MW) is dissolved in 20 ml water, cooled to 0°, and 5-300 mg of 1-cyano-4-dimethylamino pyridinium tetrafluoroborate (CDAP) is added and mixed for 30 seconds. Triethylamine (0.2 M, 0.04 ml per 5 mg CDAP) is added dropwise with vigorous stirring, and the entire reaction mixture is transferred to 80 ml of ice cold absolute ethanol containing 1 ml of 10 N HCl. The dextran precipitates, and the precipitate is removed by centrifugation at 1000 x g for 5 min at 0°, and resolubilized in 20-50 ml of 0.25 M Na-bicarbonate buffer at pH 9.0. To this mixture 600-1000 mg of PMB (either powdered or presolubilized in water) is added and stirred for 24 hours at 8°. The entire reaction mixture is then transferred to a 50,000 molecular weight cut-off dialysis tubing and dialyzed against 0.05 M pyrogen-free phosphate buffer for 6-10 days. The final dialyzed reaction mixture is measured for protein content by spectrophotometry at 208 nm absorbance or at 595 nm using the Bradford reagent (Bio Rad, Richmond, CA, USA).

Free amino groups are determined using the ninhydrin reaction, with native PMB as a control. Analysis for nitrogen and carbon content is done using a CHN Elemental Analyzer.

In the table below, "molar ratio" is determined by dividing the concentration of dextran used in conjugation (23.7 µmol) by the final PMB-protein concentration in µmol (based on 208 nm analysis) after dialysis. Dextran is abbreviated "dx". The "C:N" ratio assumes 450 molecules of water per 2 g dextran. The "bonds/PMB" is an estimate of the number of bonds by which PMB is attached to dextran, based on the ninhydrin reaction. "ND" is "not determined".

| **Summary of conjugation reactions** | | | | |
|---|---|---|---|---|
| Reaction | CDAP | molar ratio | C:N ratio | bonds/PMB |
| A | 21 µmol | 1 dx : 0.23 PMB | ND | ND |
| B | 106 µmol | 1 dx : 1.62 PMB | 1 dx : 1.52 PMB | 3.0 |
| BB | 106 µmol | 1 dx : 1.95 PMB | 1 dx : 1.90 PMB | 1.1 |
| C | 532 µmol | 1 dx : 3.70 PMB | 1 dx : 3.25 PMB | 3.0 |
| CC | 532 µmol | 1 dx : 4.99 PMB | 1 dx : 5.00 PMB | 2.2 |
| D | 1064 µmol | 1 dx : 6.62 PMB | 1 dx : 8.70 PMB | 1.7 |
| DD | 1064 µmol | 1 dx : 8.81 PMB | ND | 1.3 |
| E | 2128 µmol | 1 dx :10.9 PMB | ND | 2.1 |

### Example 2: Chemical conjugation of PMB to dextran

### (Method B; amine bond)

1.25 g dextran (79,000 or 200,000 MW) is dissolved in 20 ml of distilled water and then 0.071-0.71 g Na-periodate is added. After 1 hour at 22° the reaction mixture is transferred to a column containing DEAE A25 cationic exchange resin (Pharmacia Inc., Piscataway, NJ, USA) and the mixture is collected and pooled to a single fraction of 20-25 ml. The oxidized dextran is then mixed with 2 g PMB dissolved in 80-200 ml Na-bicarbonate or Na-borate buffer (pH 8.5-9.0) and after 60 minutes, 40 ml 0.05 - 25 % Na-borohydride either in a single treatment or in multiple treatments, each treatment lasting 30 minutes to 24 hours is added. This reaction proceeds for 30 minutes and then is dialyzed for 7-10 days at 8° against 0.05 M pyrogen-free Na-phosphate buffer. The final dialyzed reaction mixture is analyzed for protein content and free amino groups as described in Example 1.

In addition to purification by extended dialysis, several representative samples are rapidly purified using dialysis for 18 hours, purified on a G-100 Sephadex^{R} column (Pharmacia, Inc., Piscataway, NJ, USA) and concentrated on an Amicon^{R} filtration unit using a YM-100 Amicon^{R} filter.

In the table below the molar ratio is determined by dividing the amount of dextran used in conjugation (6.25 µmol) by the µmol final PMB protein (based on the 208 nm data) after dialysis. The percent nitrogen is determined using a CHN elemental analyzer. Bonds/PMB is estimated by the ninhydrin reaction:

| **Summary of conjugation reactions** | | | | |
|---|---|---|---|---|
| Reaction | NaIO₄ (g) | molar ratio | % nitrogen | bonds/PMB |
| 1/50 | 0.014 | 1 dx : 3.64 PMB | 0.21 | 3.6 |
| 1/10 | 0.071 | 1 dx : 12.9 PMB | 1.05 | 2.9 |
| 1/5 | 0.142 | 1 dx : 26.8 PMB | 1.71 | 2.6 |
| 1/4 | 0.178 | 1 dx : 29.8 PMB | 2.76 | 3.0 |
| 1/2¹ | 0.355 | 1 dx : 39.6 PMB | 3.74 | 2.2 |
| stock* | 0.710 | 1 dx :151 PMB | 6.91 | 4.0 |

| | | | | |
|---|---|---|---|---|
| *Reaction mixture is turbid and remains so after dialysis | | | | |
| ¹Purified using rapid dialysis | | | | |

### Example 3: Anti-endotoxin activity of PMB-conjugates

### A. Endotoxin-induced lethality

Male CB57BL/c mice (18-22 g, Jackson Labs) are used throughout this study. Animals are given endotoxin (0111B₄ from List Biologicals, Palo Alto, CA, USA) and galactosamine to hypersensitize, as described in Galanos et al., Proc. Natl. Acad. Sci. USA 76 (1979) 5939. The endotoxin and galactosamine (320 mg/kg) are injected intraperitoneally in 0.5 ml pyrogen-free isotonic saline per animal between 13:00 and 15:00 to avoid diurnal variation. From a dose-response study, it is determined that 0.01 mg/kg endotoxin produces 85-95 % lethality. Animals are observed each day for 6 days after injection.

### B. PMB (native and conjugated forms) induced lethality

PMB (native and conjugated forms) are evaluated for their inherent toxicity in non-sensitized mice. Male CB57BL/c mice (18-22 g, Jackson Labs) are used throughout this study. Animals are given either native PMB or conjugated PMB by intraperitoneal injection (0.5 ml/animal) and lethality is monitored for 7 days. The LD₁₀₀ is determined by varying the dose of PMB (native and conjugated forms) that result in 100 % lethality. From 10 to 20 animals are used for each dose.

### C. PMB anti-endotoxin evaluations

PMB (native and conjugated forms) are evaluated for their ability to neutralize endotoxin by either pre-mixing with endotoxin for 60 minutes before i.p. injection or by coadministration of the substances as a single i.p. injection. While the concentration of the PMB (native and conjugated forms) are varied, the volume of each injection is kept constant at 0.5 ml per animal. The Protection by the conjugated PMB is compared to the native PMB (in terms of mg protein/kg). Controls (endotoxin, galactosamine and vehicle) are included as well in each study. Each group has between 10 and 17 animals.

Statistical analyses of survival between groups or in relation to controls is performed using a Chi-square analysis using the Yate's correction for continuity.

### D. Determination of Therapeutic Index

The Therapeutic Index (TI) for PMB (native and conjugated forms) is determined by dividing the LD₁₀₀ by the PD₁₀₀. In the table below, the "treatment" refers to the conjugates appearing in Examples 1 and 2, supra.

| **Anti-endotoxin data** | | | |
|---|---|---|---|
| Treatment | LD₁₀₀ (mg/kg) | PD₁₀₀ (mg/kg) | TI |
| Native PMB¹ | 25 | 2 | 12.5 |
| Native PMB² | 25 | 2 | 12.5 |
| A | >30 | 1 | >30 |
| B | >28 | >2 | >14 |
| C | ND | 5 | ND |
| D | 140 | 2 | 70 |
| 1/5* | 83 | 0.2 | 415 |
| 1/2¹* | about 40-60 | 0.05 | 800-1200 |
| stock² | >212 | 0.2 | >1060 |
| stock³ | >212 | 20 | >10.6 |

| | | | |
|---|---|---|---|
| ¹Rapidly-purified material | | | |
| ²The PMB or conjugate is allowed to react with the endotoxin for 60 min before i.p. injection | | | |
| ³The PMB or conjugate is injected simultaneously with the endotoxin | | | |
| *The fractions designate reaction conditions based on the amount of sodium periodate used in the conjugation reaction. If 710 mg is multiplied by the fraction the amount (in mg) of sodium periodate used in each sample is obtained. | | | |

### Example 4: Ultra-pure PMB-dextran conjugate

### (Method C; alcohol precipitation)

### 1. Preparation

PMB-dextran conjugate is prepared essentially as described in Example 2, supra, to obtain conjugate designated "RXN 1/50".

The conjugate, a white flocculant, is precipitated in 60 % methanol, collected by centrifugation, and then subjected to ultrasonic resolubilization. This process is repeated seven times and the resulting material is evaluated for purity by gel permeation chromatography and reverse phase high pressure liquid chromatography (RP-HPLC). Although no free PMB is detected by gel permeation chromatography, RP-HPLC shows 48 µg unconjugated PMB per mg of total protein. The conjugate is then subjected to 3 additional precipitations to remove the unconjugated PMB.

### 2. Biological activity

The resulting ultra-pure PMB-dextran conjugate is compared to native PMB in an endotoxin-induced lethality test as described in Example 3, C., supra.

At 1 mg/kg i.v. the ultra-pure PMB-conjugate gives > 70 % protection against a LD₉₀ endotoxin challenge for at least 6 hours. At 10 mg/kg i.v. this conjugate provides therapeutic protection 1.5 hours after administration of the endotoxin. Therefore the PMB-conjugate is suitable for prophylactic use.

The ultra-pure PMB-conjugate has a surprisingly reduced toxicity as compared with native PMB. Native PMB has an LD₀ i.v. of 5 mg/kg and an LD₁₀₀ i.v. of 9.5 mg/kg, suggesting an early onset of toxicity and narrow therapeutic range. The ultra-pure PMB-conjugate has an LD₀ of > 100 mg/kg and it has not been possible to establish an LD₁₀₀ due to its inherent non-toxicity. Comparisons in the table below are made between the highest non-toxic dose of PMB (native or conjugated):

| **Biological activity** | | | |
|---|---|---|---|
| Treatment | LD₀ (mg/kg)¹ | PD₁₀₀ (mg/kg)² | TI |
| PMB | 5 | 0.1 | 50 |
| ultra-pure conjugate | > 100 | 0.1 | > 1000 |

| | | | |
|---|---|---|---|
| ¹ PMB (native or conjugate) given by i.v. injection and lethality monitored over 7 days | | | |
| ² PMB (native or conjugate) given by i.v. injection 1-3 minutes before an LD₉₀ i.p. injection of E.coli 0111B4 endotoxin (0.5 µg/kg) | | | |

## Claims (Claims for the following Contracting State(s): AT, BE, CH, DE, DK, FR, GB, IT, LI, LU, NL, SE)

1. A water-soluble conjugate of a polymyxin and a carrier, the conjugate being of a size suitable for use in the systemic circulation, with the proviso that when the polymyxin is polymyxin E then the carrier is other than a protein carrier.

2. A conjugate according to claim 1 wherein the carrier is a polysaccharide such as dextran or hydroxyethyl starch, or a polymer such as polyvinylpyrrolidone, polyethylene glycol or polyvinyl alcohol, whereby, when the polymyxin is other than polymyxin E, the carrier may also be a protein carrier such as albumin.

3. A conjugate according to claim 1 wherein the polymyxin is polymyxin B (PMB).

4. A conjugate according to claim 1 wherein the molar ratio of polymyxin B (PMB) to carrier is from 1:15 to 200:1.

5. A conjugate according to claim 1 wherein the molar ratio of polymyxin B (PMB) to carrier is from 1:1 to 10:1.

6. A conjugate according to claim 1 wherein the molar ratio of polymyxin B (PMB) to carrier is from 1:1 to 3:1.

7. A conjugate according to claim 1 having a solubility in water of at least 1 mg/ml at 25°C.

8. A conjugate according to claim 1 which is injectable.

9. A conjugate according to claim 1 which is non-matrix.

10. A conjugate according to claim 1 which is colloid.

11. A conjugate according to claim 1 which is not cross-linked.

12. A conjugate according to claim 1 which is non-macromolecular.

13. A conjugate according to claim 1 which is non-particulate.

14. A conjugate according to claim 1 wherein the carrier is uniform, non-toxic, non-carcinogenic, non-irritating and non-immunogenic.

15. A conjugate according to claim 1 wherein the carrier has a molecular weight of from 25 000 to 500 000.

16. A conjugate according to claim 1 wherein the carrier has a molecular weight of from 50 000 to 300 000.

17. A conjugate according to claim 1 wherein the carrier has a molecular weight of from 79 000 to 200 000.

18. A conjugate according to claim 1 having endotoxin-neutralizing activity.

19. A conjugate according to claim 1 having a molar ratio of conjugate to unbound polymyxin of at least 99:1.

20. A conjugate according to claim 2 wherein the carrier is dextran.

21. A conjugate according to claim 3 wherein the carrier is dextran.

22. A conjugate according to claim 21 wherein polymyxin B (PMB) is attached through carbamate linkage.

23. A conjugate according to claim 21 wherein polymyxin B (PMB) is attached through amine bonds.

24. A water-soluble conjugate of polymyxin B (PMB) and a carrier having anti-endotoxin activity, the carrier being of a size suitable for use in the systemic circulation, whereby the molar ratio of polymyxin B to carrier is from 1:15 to 200:1.

25. A conjugate according to claim 24, the carrier being of a size suitable for delivery and circulation in the blood stream.

26. A conjugate according to any one of claims 1 to 25 for use as a pharmaceutical.

27. A conjugate according to claim 26 for use in neutralizing the activity of endotoxin.

28. A process for the preparation of a polymyxin-dextran conjugate according to claim 20 comprising:
a) appropriately conjugating polymyxin to dextran to form a water-soluble polymyxin-dextran conjugate of a size suitable for use in the systemic circulation;
b) precipitating the resultant conjugate in a lower alcohol;
c) resuspending the conjugate; and
d) repeating steps b) and c) until the conjugate is substantially free from unbound polymyxin.

29. A pharmaceutical composition comprising a conjugate according to any one of claims 1 to 25 together with further pharmaceutically acceptable carriers or diluents.

30. Use of a conjugate according to any one of claims 1 to 25 for the manufacture of a medicament for prophylactic or curative therapeutic application as an endotoxin-neutralizing agent.

31. Use according to claim 30 as an antifungal agent.

32. Use according to claim 30 as an antibacterial agent.

## Claims (Claims for the following Contracting State(s): ES, GR)

1. A process for the preparation of a pharmaceutical composition comprising mixing a water-soluble conjugate of a polymyxin and a carrier together with further pharmaceutically acceptable carriers or diluents, the conjugate being of a size suitable for use in the systemic circulation, with the proviso that when the polymyxin is polymyxin E then the carrier is other than a protein carrier.

2. A process according to claim 1 wherein the carrier is a polysaccharide such as dextran or hydroxyethyl starch, or a polymer such as polyvinylpyrrolidone, polyethylene glycol or polyvinyl alcohol, whereby, when the polymyxin is other than polymyxin E, the carrier may also be a protein carrier such as albumin.

3. A process according to claim 1 wherein the polymyxin is polymyxin B (PMB).

4. A process according to claim 2 wherein the carrier is dextran.

5. A process according to claim 3 wherein the carrier is dextran.

6. A process for the preparation of a water-soluble polymyxin-dextran conjugate comprising:
a) appropriately conjugating polymyxin to dextran to form a water-soluble polymyxin-dextran conjugate of a size suitable for use in the systemic circulation;
b) precipitating the resultant conjugate in a lower alcohol;
c) resuspending the conjugate; and
d) repeating steps b) and c) until the conjugate is substantially free from unbound polymyxin.

7. A process according to claim 6 wherein the conjugate is a polymyxin B (PMB)-dextran conjugate.

8. A process according to claim 6 wherein steps b) and c) are repeated at least three times.

9. A process for the preparation of a polymyxin B (PMB)-dextran conjugate comprising:
a) appropriately conjugating polymyxin B (PMB) to dextran to form a water-soluble polymyxin B (PMB) - dextran conjugate comprising a molecular ratio of PMB to dextran from 1:15 to 200:1 and of a size suitable for delivery and circulation in the blood stream;
b) precipitating the resultant conjugate in a lower alcohol;
c) resuspending the conjugate; and
d) repeating steps b) and c) until the conjugate is substantially free from unbound polymyxin B.

10. A process according to claim 9 wherein steps b) and c) are repeated at least three times.

## Patentansprüche (Patentansprüche für folgende(n) Vertragsstaat(en): AT, BE, CH, DE, DK, FR, GB, IT, LI, LU, NL, SE)

1. Wasserlösliches Konjugat eines Polymyxins und eines Trägers, wobei das Konjugat eine Größe aufweist, die für die Verwendung im systemischen Kreislauf geeignet ist, mit der Maßgabe, daß, wenn das Polymyxin Polymyxin E ist, der Träger dann kein Proteinträger ist.

2. Konjugat nach Anspruch 1, worin der Träger ein Polysaccharid ist, wie Dextran oder Hydroxyethylstärke, oder ein Polymer, wie Polyvinylpyrrolidon, Polyethylenglycol oder Polyvinylalkohol, wobei, wenn das Polymyxin ein anderes als Polymyxin E ist, der Träger auch ein Proteinträger, wie Albumin, sein kann.

3. Konjugat nach Anspruch 1, worin das Polymyxin Polymyxin B (PMB) ist.

4. Konjugat nach Anspruch 1, worin das Molverhältnis von Polymyxin B (PMB) zum Träger 1:15 bis 200:1 ist.

5. Konjugat nach Anspruch 1, worin das Molverhältnis von Polymyxin B (PMB) zum Träger 1:1 bis 10:1 ist.

6. Konjugat nach Anspruch 1, worin das Molverhältnis von Polymyxin B (PMB) zum Träger 1:1 bis 3:1 ist.

7. Konjugat nach Anspruch 1, das eine Löslichkeit in Wasser von mindestens 1 mg/ml bei 25°C aufweist.

8. Konjugat nach Anspruch 1, das injizierbar ist.

9. Konjugat nach Anspruch 1, das nicht matrixartig ist.

10. Konjugat nach Anspruch 1, das kolloidal ist.

11. Konjugat nach Anspruch 1, das nicht quervernetzt ist.

12. Konjugat nach Anspruch 1, das nicht makromolekular ist.

13. Konjugat nach Anspruch 1, das nicht partikulär ist.

14. Konjugat nach Anspruch 1, worin der Träger einheitlich, nicht toxisch, nicht carcinogen, nicht reizend und nicht immunogen ist.

15. Konjugat nach Anspruch 1, worin der Träger ein Molekulargewicht von 25 000 bis 500 000 aufweist.

16. Konjugat nach Anspruch 1, worin der Träger ein Molekulargewicht von 50 000 bis 300 000 aufweist.

17. Konjugat nach Anspruch 1, worin der Träger ein Molekulargewicht von 79 000 bis 200 000 aufweist.

18. Konjugat nach Anspruch 1, das Endotoxin-neutralisierende Aktivität aufweist.

19. Konjugat nach Anspruch 1, das ein Molverhälnis von Konjugat zu ungebundenem Polymyxin von mindestens 99:1 aufweist.

20. Konjugat nach Anspruch 2, worin der Träger Dextran ist.

21. Konjugat nach Anspruch 3, worin der Träger Dextran ist.

22. Konjugat nach Anspruch 21, worin Polymyxin B (PMB) durch eine Carbamatbindung gebunden ist.

23. Konjugat nach Anspruch 21, worin Polymyxin B (PMB) durch Aminbindungen gebunden ist.

24. Wasserlösliches Konjugat von Polymyxin B (PMB) und einem Träger mit Anti-Endotoxin Aktivität, wobei der Träger eine für die Verwendung im systemischen Kreislauf geeignete Größe hat und das Molverhältnis von Polymyxin B zum Träger 1:15 bis 200:1 beträgt.

25. Konjugat nach Anspruch 24, wobei der Träger für die Angabe und Zirkulation in den Blutstrom geeignet ist.

26. Konjugat nach einem der Ansprüche 1 bis 25 zur Verwendung als Pharmazeutikum.

27. Konjugat nach Anspruch 26 zur Verwendung bei der Neutralisierung der Endotoxinwirkung.

28. Verfahren zur Herstellung eines Polymyxin-Dextran-Konjugats nach Anspruch 20, gekennzeichnet durch
a) geeignete Konjugation von Polymyxin an Dextran unter Bildung eines wasserlöslichen Polymyxin-Dextran-Konjugats einer Größe, die zur Verwendung im systemischen Kreislauf geeignet ist,
b) Fällung des entstehenden Konjugats in einem niederen Alkohol,
c) Resuspendierung des Konjugats, und
d) Wiederholung der Schritte b) und c), bis das Konjugat im wesentlichen frei von ungebundenem Polymyxin ist.

29. Pharmazeutische Zusammensetzung, die ein Konjugat nach einem der Ansprüche 1 bis 25 zusammen mit weiteren pharmazeutisch annehmbaren Trägern oder Verdünnungsmitteln enthält.

30. Verwendnung eines Konjugats nach einem der Ansprüche 1 bis 25 zur Herstellung eines Arzneimittels zur prophylaktischen oder heilenden therapeutischen Anwendung als Endotoxin-neutralisierendes Mittel.

31. Verwendung nach Anspruch 30 als antifungales Mittel.

32. Verwendung nach Anspruch 30 als antibakterielles Mittel.

## Patentansprüche (Patentansprüche für folgende(n) Vertragsstaat(en): ES, GR)

1. Verfahren zur Herstellung einer pharmazeutischen Zusammensetzung, gekennzeichnet durch Mischen eines wasserlöslichen Konjugats eines Polymyxins und eines Trägers mit weiteren pharmazeutisch annehmbaren Trägern oder Verdünnungsmitteln, wobei das Konjugat eine Größe aufweist, die zur Verwendung im systemischen Kreislauf geeignet ist, mit der Maßgabe, daß, wenn das Polymyxin Polymyxin E ist, der Träger dann kein Proteinträger ist.

2. Verfahren nach Anspruch 1, worin der Träger ein Polysaccharid ist, wie Dextran oder Hydroxyethylstärke, oder ein Polymer, wie Polyvinylpyrrolidon, Polyethylenglycol oder Polyvinylalkohol, wobei, wenn das Polymyxin ein anderes als Polymyxin E ist, der Träger auch ein Proteinträger, wie Albumin, sein kann.

3. Verfahren nach Anspruch 1, worin das Polymyxin Polymyxin B (PMB) ist.

4. Verfahren nach Anspruch 2, worin der Träger Dextran ist.

5. Verfahren nach Anspruch 3, worin der Träger Dextran ist.

6. Verfahren zur Herstellung eines wassserlöslichen Polymyxin-Dextran-Konjugats, gekennzeichnet durch
a) geeignete Konjugation von Polymyxin an Dextran unter Bildung eines wasserlöslichen Polymyxin-Dextran-Konjugats einer Größe, die zur Verwendung im systemischen Kreislauf geeignet ist,
b) Fällung des entstehenden Konjugats in einem niederen Alkohol,
c) Resuspendierung des Konjugats, und
d) Wiederholung der Schritte b) und c), bis das Konjugat im wesentlichen frei von ungebundenem Polymyxin ist.

7. Verfahren nach Anspruch 6, worin das Konjugat ein Polymyxin B (PMB)-Dextran-Konjugat ist.

8. Verfahren nach Anspruch 6, worin die Schritte b) und c) mindestens dreimal wiederholt werden.

9. Verfahren zur Herstellung eines Polymyxin B (PMB)-Dextran-Konjugats, gekennzeichnet durch
a) geeignete Konjugation von Polymyxin B (PMB) an Dextran unter Bildung eines wasserlöslichen Polymyxin B (PMB)-Dextran-Konjugats mit einem Molverhältnis von PMB zu Dextran von 1:15 bis 200:1 und einer Größe, die zur Abgabe und Zirkulation in den Blutstrom geeignet ist,
b) Fällung des entstehenden Konjugats in einem niederen Alkohol,
c) Resuspendierung des Konjugats, und
d) Wiederholung der Schritte b) und c), bis das Konjugat im wesentlichen frei von ungebundenem Polymyxin B ist.

10. Verfahren nach Anspruch 9, worin die Schritte b) und c) mindestens dreimal wiederholt werden.

## Revendications (Revendications pour l'(les) Etat(s) contractant(s) suivant(s): AT, BE, CH, DE, DK, FR, GB, IT, LI, LU, NL, SE)

1. Un conjugé soluble dans l'eau d'une polymyxine et d'une molécule porteuse, le conjugué étant d'une dimension appropriée pour l'utilisation dans la circulation systémique, la molécule porteuse devant être autre qu'une protéine lorsque la polymyxine est la polymyxine E.

2. Un conjugué selon la revendication 1, dans lequel la molécule porteuse est un polysaccharide tel que le dextrane ou l'hydroxyéthylamidon, ou un polymère tel que la polyvinylpyrrolidone, le polyéthylèneglycol ou l'alcool polyvinylique, la molécule porteuse pouvant être également une protéine telle que l'albumine lorsque la polymyxine est autre que la polymyxine E.

3. Un conjugué selon la revendication 1, dans lequel la polymyxine est la polymyxine B(PMB).

4. Un conjugué selon la revendication 1, dans lequel le rapport molaire de la polymyxine B(PMB) à la molécule porteuse est de 1:15 à 200:1.

5. Un conjugué selon la revendication 1, dans lequel le rapport molaire de la polymyxine a (PMB) à la molécule porteuse est de 1:1 à 1O:1.

6. Un conjugué selon la revendication 1, dans lequel le rapport molaire de la polymyxine B (PMB) à la molécule porteuse est de 1:1 à 3:1.

7. Un conjugué selon la revendication 1, ayant une solubilité dans l'eau d'au moins 1 mg/ml à 25°C.

8. Un conjugué selon la revendication 1, qui est injectable.

9. Un conjugué selon la revendication 1, qui n'est pas matriciel.

10. Un conjugué selon la revendication 1, qui est un colloïde.

11. Un conjugué selon la revendication 1, qui n'est pas réticulé.

12. Un conjugué selon la revendication 1, qui n'est pas macromoléculaire.

13. Un conjugué selon la revendication 1, qui n'est pas sous forme de particules.

14. Un conjugué selon la revendication 1, dans lequel la molécule porteuse est uniforme, non toxique, non carcinogène, non irritante et non immunogène.

15. Un conjugué selon la revendication 1, dans lequel la molécule porteuse a un poids moléculaire de 25.000 à 50.000.

16. Un conjugué selon la revendication 1, dans lequel la molécule porteuse a un poids moléculaire de 50.000 à 300.000.

17. Un conjugué selon la revendication 1, dans lequel la molécule porteuse a un poids moléculaire de 79.000 à 200.000.

18. Un conjugué selon la revendication 1, ayant une activité neutralisant l'endotoxine.

19. Un conjugué selon la revendication 1, ayant un rapport molaire du conjugué à la polymyxine non couplée d'au moins 99:1.

20. Un conjugué selon la revendication 2, dans lequel la molécule porteuse est le dextrane.

21. Un conjugué selon la revendication 3, dans lequel la molécule porteuse est le dextrane.

22. Un conjugué selon la revendication 21, dans lequel la polymyxine B (PMB) est couplée par une liaison carbamate.

23. Un conjugué selon la revendication 21, dans lequel la polymyxine B (PMB) est couplée par des liaisons amino.

24. Un conjugué soluble dans l'eau de polymyxine B (PMB) et d'une molécule porteuse ayant une activité anti-endotoxine, la molécule porteuse étant d'une dimension appropriée pour l'utilisation dans la circulation systémique, le rapport molaire de la polymyxine B à la molécule porteuse étant de 1:15 à 200:1.

25. Un conjugué selon la revendication 24, la molécule porteuse étant d'une dimension appropriée pour l'apport et la circulation dans le courant sanguin.

26. Un conjugué selon l'une quelconque des revendications 1 à 25 pour l'utilisation comme médicament.

27. Un conjugué selon la revendication 26 destiné à être utilisé pour neutraliser l'activité de l'endotoxine.

28. Un procédé pour la préparation d'un conjugué polymyxine-dextrane selon la revendication 20, comprenant :
a) le couplage d'une manière appropriée de la polymyxine au dextrane pour former un conjugué polymyxine-dextrane soluble dans l'eau et d'une dimension appropriée pour l'utilisation dans la circulation systémique;
b) la précipitation du conjugué résultant dans un alcool inférieur;
c) la remise en suspension du conjugué; et
d) la répétition des étapes b) et c) jusqu'à ce que le conjugué soit substantiellement exempt de polymyxine non couplée.

29. Une composition pharmaceutique comprenant un conjugué selon l'une quelconque des revendications 1 à 25, en association avec des véhicules ou diluents pharmaceutiquement acceptables.

30. L'utilisation d'un conjugué selon l'une quelconque des revendications 1 à 25, pour la préparation d'un médicament destiné à l'application thérapeutique prophylactique ou curative comme agent neutralisant l'endotoxine.

31. L'utilisation selon la revendication 30 comme agent antifongique.

32. L'utilisation selon la revendication 30 comme agent antibactérien.

## Revendications (Revendications pour l'(les) Etat(s) contractant(s) suivant(s): ES, GR)

1. Un procédé de préparation d'une composition pharmaceutique comprenant le mélange d'un conjugé soluble dans l'eau d'une polymyxine et d'une molécule porteuse, avec des véhicules ou diluents pharmaceutiquement acceptables, le conjugué étant d'une dimension appropriée pour l'utilisation dans la circulation systémique, la molécule porteuse devant être autre qu'une protéine lorsque la polymyxine est la polymyxine E.

2. Un procédé selon la revendication 1, dans lequel la molécule porteuse est un polysaccharide tel que le dextrane ou l'hydroxyéthylamidon, ou un polymère tel que la polyvinylpyrrolidone, le polyéthylèneglycol ou l'alcool polyvinylique, la molécule porteuse pouvant être également une protéine telle que l'albumine lorsque la polymyxine est autre que la polymyxine E.

3. Un procédé selon la revendication 1, dans lequel la polymyxine est la polymyxine B (PMB).

4. Un procédé selon la revendication 2, dans lequel la molécule porteuse est le dextrane.

5. Un procédé selon la revendication 3, dans lequel la molécule porteuse est le dextrane.

6. Un procédé de préparation d'un conjugué soluble dans l'eau polymyxine-dextrane, comprenant :
a) le couplage d'une manière appropriée de la polymyxine au dextrane pour former un conjugué polymyxine-dextrnae soluble dans l'eau et d'une dimension appropriée pour l'utilisation dans la circulation systémique;
b) la précipitation du conjugué résultant dans un alcool inférieur;
c) la remise en suspension du conjugué; et
d) la répétition des étapes b) et c) jusqu'à ce que le conjugué soit substantiellement exempt de polymyxine non couplée.

7. Un procédé selon la revendication 6, dans lequel le conjugué est un conjugué polymyxine B (PMB)-dextrane.

8. Un procédé selon la revendication 6, dans lequel les étapes b) et c) sont répétées au moins trois fois.

9. Un procédé de préparation d'un conjugué polymyxine B (PMB)-dextrane comprenant :
a) le couplage d'une façon appropriée de la polymyxine B (PMB) au dextrane pour former un conjugué soluble dans l'eau polymyxine B (PMB)-dextrane ayant un rapport moléculaire de PMB au dextrane de 1:15 à 200:1 et d'une dimension appropriée pour l'apport et la circulation dans le courant sanguin ;
b) la précipitation du conjugué résultant dans un alcool inférieur ;
c) la remise en suspension du conjugué; et
d) la répétition des étapes b) et c) jusqu'à ce que le conjugué soit substantiellement exempt de polymyxine B non couplée.

10. Un procédé selon la revendication 9, dans lequel les étapes b) et c) sont répétées au moins trois fois.
